# EUROPEAN PATENT APPLICATION

(11) **EP 0 579 868 A2**
(43) Date of publication of application: **26.01.1994**
(21) Application number: 92120122.4
(22) Date of filing: 25.11.1992
(51) Int. Cl.: A61F 2/36, A61F 2/30

(54) **Femoral rod for a complete hip prosthesis**

(30) Priority: 24.07.1992 IT TO920634
(71) Applicant: HOWMEDICA INTERNATIONAL Div.ne PFIZER ITALIANA S.p.A., I-04100 Borgo San Michele-Latina (IT)
(72) Inventor: Calderale, Pasquale Mario, 10129 Torino (IT); Pipino, Francesco, 70100 Bari (IT)
(74) Representative: Jorio, Paolo

(57) **Abstract**

A femoral rod (1) comprising a shank (2) inserted inside the femur; a head (5) for the articulation of the femur; and means (9) for transferring the physiological loads normally supported by the femur on to a section (11) of the femoral neck (10) immediately below the head of the femur; which means consist of a transversely projecting collar (13) at the proximal end of the shank (2), of substantially the same external shape and size as the neck (10) below the head of the femur.

## Description

The present invention relates to a femoral rod for a complete hip prosthesis.

Complete hip prostheses currently feature a rod consisting of a shank inserted inside the femur, and a spherical head projecting from the bone and cooperating with an acetabular cup in turn fitted to the periacetabular bone.

To insert the rod, a resection is effected at the base of the neck to remove the head and neck of the femur, and the shank of the rod is actually forced or cemented in place inside the medullary canal. To ensure the vertical stability of the rod, the proximal or top end facing the head sometimes presents a small flange for preventing the rod from subsiding inside the femur.

Femoral rods of the aforementioned type are normally designed to be press fitted inside the medullary canal, so as to provide for a tight fit between the rod and the cortical bone, for which purpose the spongy bone inside the medullary canal is normally removed.

Known rods and the method whereby they are inserted present several drawbacks.

Firstly, removal of the nondiseased neck of the femur, which is rich in spongy bone, seriously affects normal load transmission, which is extremely difficult to adapt to.

Secondly, by virtue of the shank being press fitted tightly inside the medullary canal, the elasticity of the resulting structure varies sharply and, hence, discontinuously at the prosthesis-bone interface, thus further affecting normal load transmission, and preventing the bone from adapting to a more physiological condition wherein elasticity varies transversely in relation to the bone.

Thirdly, in the event of failure, removal of the rod poses serious problems, due to the difficulty of separating it from the attached bone.

Lastly, the shape and size of the distal or end portion of the shank may result in what is commonly referred to as the "tip effect" or undesired embedding of the distal portion, thus resulting in hypertrophy of the cortical bone or at any rate painful irritation of the bone, and possibly also in detachment of the rod from the bone as of the proximal or top portion of the rod.

It is an object of the present invention to provide a rod designed to overcome the aforementioned drawbacks, while at the same time respecting normal biological and biomechanical conditions.

According to the present invention, there is provided a femoral rod for a complete hip prosthesis, comprising a shank inserted inside the femur, and a head for the articulation of the femur; characterized by the fact that it comprises means for transferring the physiological loads normally supported by the femur on to a section of the neck immediately below the head of the femur.

According to a preferred embodiment of the present invention, said means consist of a transversely projecting collar at the proximal or top end of the shank, and of substantially the same external shape and size as the neck below the head of the femur.

According to the present invention, there is also provided a system enabling articulated connection of the periacetabular bone and the femur, characterized by the fact that it comprises:
a femoral rod substantially comprising a shank inserted inside the femur; a head extending axially from said shank; and a collar projecting transversely from and located at the proximal end of said shank;
an acetabular cup fitted to the periacetabular bone, for housing and enabling angular oscillation of the rod in relation to the periacetabular bone;
a femoral neck projecting integrally from the femur, surrounding a portion of said shank adjacent to said proximal end, and designed to support said collar of said rod;
the axial length of the femoral neck being substantially equal to that produced by a resection effected below the head of the femur;
the size of said collar of said rod, measured in directions lying in the plane defined by said resection, being substantially equal to the thickness of the femoral neck measured in the same directions.

In other words, insertion of the shank of the rod according to the present invention requires that the neck of the femur be retained, thus enabling the spongy bone inside the neck and the proximal metaphysis to be employed for cushioning load and so producing a variably elastic structure. The collar of the rod provides for transmitting load physiologically on to the neck of the femur, and for arresting potential longitudinal movement of the shank as described in detail later on.

Moreover, by virtue of the shape and size of the rod as described later on, the rod according to the present invention provides for transmitting load along the lines of force in the physiological direction, while the extremely short length of the rod provides for reliable, troublefree insertion, and perfect centering of the bottom end or distal portion of the rod inside the femoral cavity. Finally, in the event of failure of the prosthesis according to the present invention, the head may be resected for inserting a conventional rod, in which case, removal and replacement of the rod according to the present invention are greatly facilitated.

A preferred non-limiting embodiment of the present invention will be described by way of example with reference to the accompanying drawings, in which:
Fig.1 shows a front view of the rod according to the present invention;
Fig.2 shows a side view of the Fig.1 rod;
Fig.3 shows a further schematic front view of the rod;
Fig.4 shows a view of the rod as inserted inside the femur;
Fig.5 shows a top view of the Fig.4 rod-femur assembly;
Fig.s 6 to 11 show design variations of the end or distal portion of the rod;
Fig.12 shows a schematic view of the rod-acetabular cup connection of a complete hip prosthesis.

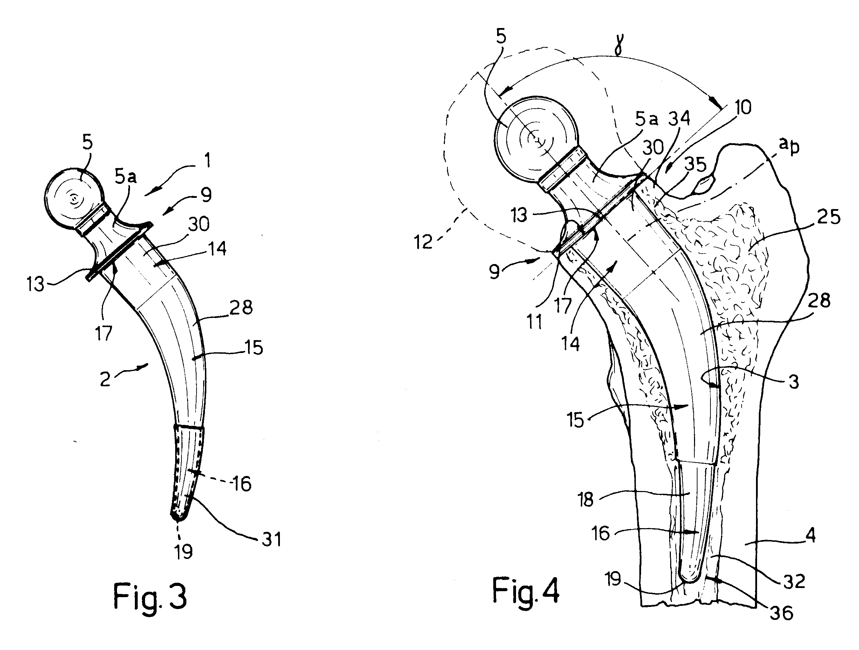

With reference to Fig.s 1 and 2, number 1 indicates a femoral rod substantially comprising a shank 2 inserted inside the medullary cavity 3 (Fig.4) of femur 4; and a head 5 inserted inside the cavity 6 (Fig.12) of a cup 7 inserted inside the acetabular cavity 8 of the hip, as shown in Fig.12, and which may be a biequatorial cup as described in Italian utility patent n.1095649.

According to the present invention, rod 1 substantially comprises means 9 for transferring the physiological loads normally supported by femur 4 on to section 11 of femoral neck 10 (Fig. 4) just below the head 12 of the femur (shown by the dotted line in Fig.4).

Means 9 consist substantially of a transversely projecting collar 13 at the top end of shank 2, of substantially the same external shape and size as neck 10 beneath head 12 (Fig. 4), and connected to head 5 by a connecting portion 5a.

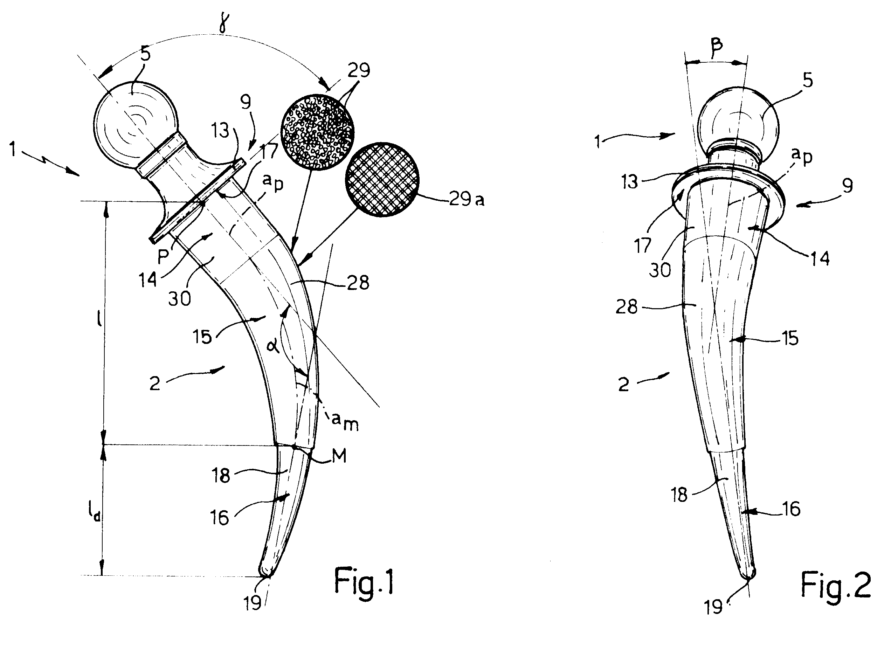

According to the present invention, shank 2 substantially comprises a proximal or top portion 14 just below collar 13 and having a substantially straight axis aₚ (Fig.1); a mid portion 15 adjacent to portion 14 and having a curved axis aₘ blending with axis aₚ; and a distal or bottom portion 16.

Conveniently, though not necessarily, proximal portion 14 tapers slightly towards the bottom end of shank 2.

With reference to the front view shown in Fig.1, axis aₚ of proximal portion 14 forms an angle α of 125-130° with a line tangent to curved axis aₘ of mid portion 15 at end point M; and the distance "l" between point M and point P, at which axis aₚ intersects the bottom surface 17 of collar 13, ranges from 80 to 120 mm.

The length of proximal portion 14 conveniently ranges from 10 to 20 mm.

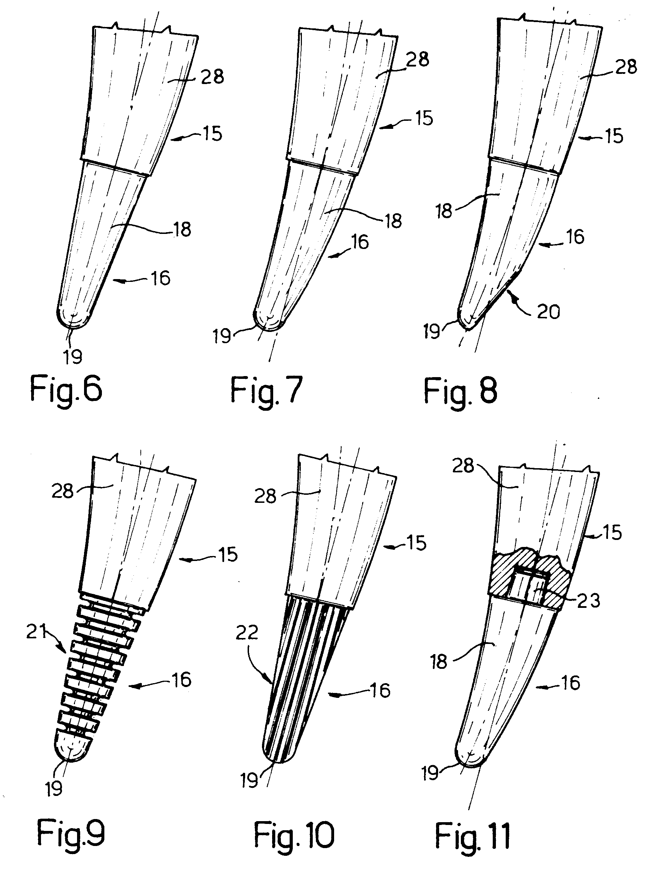

Distal portion 16 presents a smooth surface 18; a length l_{d} ranging from 0.33 to 0.50 of the total length of shank 2; a rounded tip 19; and may be formed as shown in any of Fig.s 6 to 11: with a straight or curved axis (Fig.s 6 and 7); with a bevel 20 on the convex side of surface 18 (Fig.8); with a number of circumferential grooves 21 (Fig. 9) or axial grooves 22 (Fig. 10) for increasing flexibility as compared with the rest of shank 2, for the reasons described later on; or from different material as compared with the rest of shank 2, e.g. composite synthetic material, in which case, it conveniently presents a projection 23 for insertion inside a cavity on mid portion 15 (Fig. 11).

The rod according to the present invention also presents the curves shown in the Fig.2 sagittal plane perpendicular to the front plane in Fig.1. In the Fig.2 plane, axis aₚ of proximal portion 14 forms an angle β of 10-30° with the axis of the rest of shank 2, said axes blending as shown in Fig.2; and mid portion 15 may conveniently be slightly buckled.

Bottom surface 17 of collar 13 (Fig.1) forms an angle τ of 75-90° (90° in the embodiment shown) with axis aₚ of proximal portion 14.

Surface 28 externally defining proximal portion 14 and mid portion 15 (Fig.s 1 and 2) presents means for assisting osteointegration with spongy bone 25 (Fig.4), for which purpose, surface 28 presents projections 29 (enlarged detail in Fig.1) ranging in average size from 0.5 to 1.5 mm, and conveniently also provided on bottom surface 17 of collar 13.

Alternatively, surface 28 presents a 1-2.5 mm mesh net sheath 29a (enlarged detail in Fig.1).

Proximal portion 14 conveniently presents a hydroxylapatite coating 30 (Fig.3) for assisting osteointegration of the shank surface and the spongy bone of the neck and femoral cavity; and distal portion 16 presents a sheath 31 of highly elastic, absorbable, biodegradable material for centering the tip of rod 1 during and immediately following insertion, and subsequently forming an annular gap 32 (Fig.4) between distal portion 16 and the medullary canal following absorption and breakdown of sheath 31.

Head 5 of rod 1 is preferably not integral with shank 2, connecting portion 5a presenting a conical projection (not shown) designed to fit inside a cavity in shank 2 for enabling the formation of rods 1 featuring heads and shanks of different sizes and materials.

Rod 1 according to the present invention is inserted as follows.

The first stage consists in resecting head 12 of femur 4 (Fig.4) at section 11 just below head 12, which section is so selected as to preserve a portion of neck 10 with an annular portion of cortical bone 34 and the corresponding spongy bone 35. The axial length of said neck portion is so selected as to maintain a substantially efficient blood supply and so prevent possible necrosis of neck 10.

The second stage consists in inserting rod 1 inside femoral cavity 3 so that collar 13 rests on section 11. Being substantially the same size as neck 10, collar 13 rests on neck 10 in such a manner as to cover the whole of section 11.

Following resection of head 12 in said first stage, the portions of spongy bone 35 and 25 inside neck 10 and cavity 3 are conveniently adjusted and compacted so that the inner surface of the spongy bone substantially matches the surface of shank 2, and so that, when rod 1 is inserted inside cavity 3, projections 29 (Fig.1) or net 29a penetrate gently inside the spongy bone without inducing stress in cortical bone portion 34 of neck 10.

Prior to inserting rod 1, resection surface 11 is also conveniently smoothed.

The shape and size of shank 2 provide for troublefree insertion of rod 1 inside cavity 3, distal portion 16 in fact acting as a guide during insertion, and the particular configuration of the axes in the front and sagittal planes enabling rod 1 to be adapted to the shape and size of cavity 3. Once rod 1 is inserted fully inside cavity 3, with collar 13 resting on surface 11, the size of shank 2 is such that only a small portion of distal portion 16, ranging between 20 and 30 mm in length, is inserted inside diaphyseal portion 36 (Fig.4). Consequently, surface 28 of the mid and proximal portions is arranged firmly contacting spongy bone 25, thus enabling effective integration of surface 28 and spongy bone 25 by virtue of projections 29 or net 29a on surface 28.

Moreover, annular gap 32 between distal portion 16 and the inner surface of femoral cavity 3 prevents any possibility of portion 16 contacting and knitting with the cortical bone and so resulting in an excessively stable, rigid connection of distal portion 16, in turn resulting in oscillation of rod 1 about said connecting portion.

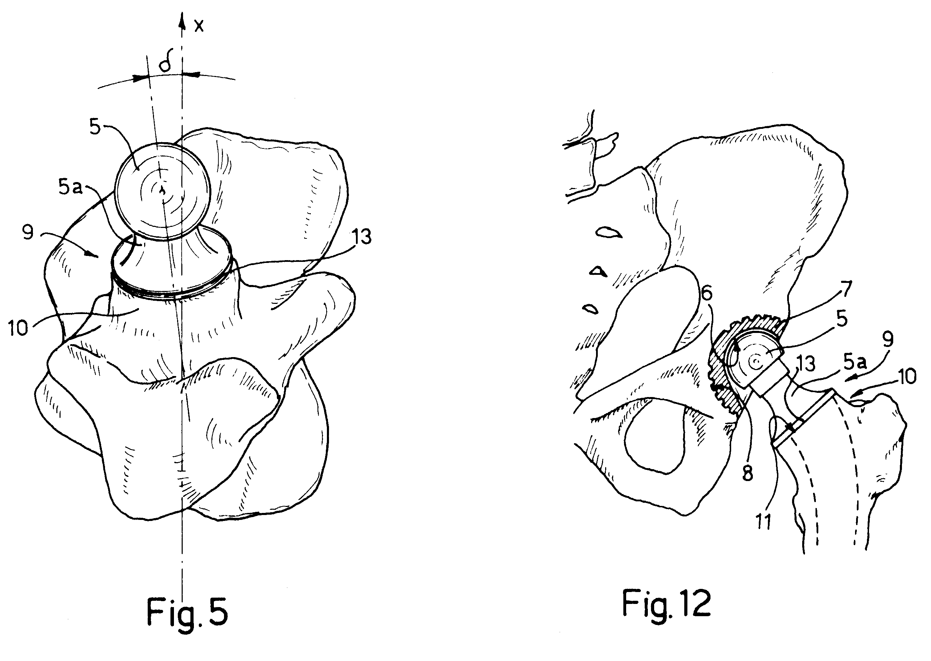

The three-dimensional design of rod 1, in particular the configuration of the axes in the front and sagittal planes, provides for strictly defining the insertion position of the rod inside the femoral cavity, and in particular the angular position of head 5 in relation to the femur. As shown in Fig.5, which shows a top view of the femur subsequent to insertion of rod 1, the center of head 5 lies along a half line forming a specific angle δ with a theoretical reference axis X integral with the femur, which angle matches the natural angle between the femur and hip, and, following insertion of rod 1 inside the hip, is strictly defined by virtue of the above geometric parameters.

The advantages of rod 1 according to the present invention will be clear from the foregoing description.

In particular, it provides for an integrated mechanical assembly involving minimum bone removal, the bone structure being preserved and closed about the rod; the shape and size of the rod are such as to preserve the metaphyseal spongy bone, and to condition the continual rearrangement occurring naturally in the spongy bone and which provides for maintaining an optimum relationship between the rod and bone; and enhanced mechanical performance of the rod and bone is achieved by providing for a system of gradually varying elasticity between the rod and the spongy and cortical bones.

Such advantages are achieved by virtue of collar 13 resting on the entire surface of section 11 of neck 10, and by virtue of the shape, size and surface finish of collar 13 and the rod, which are such as to occupy the whole of and mate with the inner surface of neck 10. The resistance and rigidity of neck 10, which is preserved entirely even after removal of head 12, enable unique proximal fitment of rod 1 as compared with known rods, which, being fitted to the metaphyseal portion of the femur as opposed to the neck, provide for transmitting load transversely along the rod at the metaphyseal spongy bone and diaphyseal cortical bone.

The rod according to the present invention provides for minimizing remodelling of the top portion of the femur following insertion.

By virtue of mating perfectly with the bone, the rod according to the present invention minimizes three-dimensional relative movement between the rod and bone, and ensures maximum primary or initial stability by providing for a high degree of torsional, inward-outward rotational and vertical stability. As regards torsional stability, the particular configuration and inclination of the shank axes in the front and sagittal planes prevent pathological microrotation of the rod inside the femur, as occurs at times inside the diaphysis in the case of conventional prostheses, all of which in fact are subject to rotational instability by virtue of requiring removal of neck 10.

As regards inward-outward rotational stability, the curvature of the rod axes and the contact surface between the rod and spongy bone and between collar 13 and surface 11 prevent the rod from rotating inwards or outwards in the front plane; which stability is also greatly enhanced by reducing the torque on head 5 in relation to cortical bone 34 of neck 10 supporting collar 13, by reducing the distance (lever arm) between head 5 and the cortical bone-rod interface (collar 13) as compared with known prostheses.

Vertical stability, on the other hand, is assured by collar 13 resting on cortical bone 34, which acts in fact as a ring for preventing the rod from subsiding inside the bone; and by the shape and characteristic angles of the rod.

In view of the high degree of initial stability provided for by the rod according to the present invention, this need no longer be press-fitted but simply inserted inside spongy bone 25, 35 as already described, thus providing for a high degree of integration of the rod and spongy bone, by virtue of the remodelling capacity of the latter.

Moreover, the short length of the rod and the fact that shank 2 is inserted inside spongy bone 25, 35 provide for a system of gradually variable elasticity which, in addition to a closer physiological resemblance, provides for absorbing stress between the prosthesis and femur, in particular, shearing stress which, in the case of discontinuously variable elasticity, could result in detachment of the rod from the bone.

The rod according to the present invention also provides for load distribution along the physiological lines of the bone, and thus for more natural performance of the bone-prosthesis system.

The design of the rod and in particular the rough finish of rod surface 28 and collar surface 17 enable purely metaphyseal fitment of the rod, with distal portion 16 freely suspended inside medullary canal 3. This is important for preventing undesired embedding of the distal portion, which is also prevented by so designing the distal portion, as shown in any one of the embodiments in Fig.s 6 to 11, that, even in the event of minor displacement of the rod in relation to the femur, no contact occurs between the distal portion and medullary canal 3, or, at most, the distal portion rests only lightly on the canal by virtue of the high degree of elasticity of distal portion 16.

As already stated, by virtue of the short length of the rod according to the present invention, only a short portion of distal portion 16 is inserted inside diaphyseal portion 36, so that the boundary section between the femur-prosthesis structure and the femur itself is located much higher (40-80 mm) as compared with conventional prostheses, and, what is more, in a portion of the bone having a highly resistant and undoubtedly more elastic and extensive section, thus greatly reducing discomfort, as compared with known prostheses, following insertion of the rod.

In the event of failure of the prosthesis, neck 10 and the short length of the rod provide for troublefree removal, by simply removing neck 10 to expose the underlying rod portion, which is sufficient for easily extracting the remainder of the rod. This may then be replaced by a conventional rod requiring removal of neck 10.

To those skilled in the art it will be clear that changes may be made to the rod as described and illustrated herein without, however, departing from the scope of the present invention.

In particular, instead of being formed whole as described herein, collar 13 may present a number of portions projecting radially from shank 2, or any other design featuring means for transferring the physiological loads normally supported by the femur on to a section of neck 10 located immediately below head 12.

Also, hydroxylapatite coating 30 and/or biodegradable, absorbable sheath 31 may be dispensed with, and changes made to the manner in which a stable connection is achieved between the rod and bone. For example, instead of a rough surface finish, surface 28 of proximal portion 14 and mid portion 15 may present a smooth surface finish, with a number of holes or indentations for assisting attachment and fitment of the rod to the metaphyseal spongy bone. Alternatively, the surface of proximal portion 14 may be grooved, scored or otherwise roughened for assisting integration and so achieving a stable connection of the rod and bone.

The present invention also relates to a system enabling articulated connection of the periacetabular bone and the femur, characterized by the fact that it comprises:
a femoral rod 1 substantially comprising a shank 2 inserted inside the femur; a head 5 extending axially from shank 2; and a collar 13 projecting transversely from and located at the top end of shank 2;
an acetabular cup 6 fitted to the periacetabular bone, for housing head 5 of rod 1 and enabling angular oscillation of the rod in relation to the periacetabular bone;
a femoral neck 10 projecting integrally from the femur, surrounding a portion of shank 2 adjacent to said top end, and designed to support said collar.

In the above system, the axial length of neck 10 is selected substantially equal to that produced by a resection effected below head 12 of the femur; and the size of collar 13, measured in directions lying in the plane defined by said resection, is substantially equal to the thickness of neck 10 measured in the same directions.

## Claims

1. A femoral rod (1) for a complete hip prosthesis, comprising a shank (2) inserted inside the femur (4), and a head (5) for the articulation of the femur (4); characterized by the fact that it comprises means (9) for transferring the physiological loads normally supported by the femur (4) on to a section (11) of the neck (10) immediately below the head of the femur.

2. A rod as claimed in Claim 1, characterized by the fact that said means (9) consist of a transversely projecting collar (13) at the top end of said shank (2), of substantially the same external shape and size as the neck (10) below the head of the femur.

3. A rod as claimed in Claim 1 or 2, characterized by the fact that said shank (2) comprises a proximal or top portion (14) immediately below said collar (13) and having a substantially straight axis aₚ; a mid portion (15) adjacent to said proximal portion (14) and having a curved axis aₘ blending with said axis aₚ; and a distal or bottom portion (16).

4. A rod as claimed in Claim 3, characterized by the fact that said proximal portion (14) tapers slightly towards the bottom end of said shank (2).

5. A rod as claimed in one of the foregoing Claims, characterized by the fact that, in the front plane, said axis aₚ of said proximal portion (14) forms an angle of 125-130° with a line tangent to end point M of said curved axis aₘ of said mid portion (15); and the distance between said point M and the point P at which said straight axis aₚ intersects the bottom surface (17) of said collar (13) ranges from 80 to 120 mm.

6. A rod as claimed in one of the foregoing Claims, characterized by the fact that said proximal portion (14) of said shank (2) presents a length of 10 to 20 mm.

7. A rod as claimed in one of the foregoing Claims, characterized by the fact that the surface (18) of said distal portion (16) is smooth; said distal portion (16) presenting a length ranging from 0.33 to 0.50 of the total length of said shank (2) and having a rounded tip (19).

8. A rod as claimed in Claim 7, characterized by the fact that the axis of said distal portion (16) is straight.

9. A rod as claimed in one of the foregoing Claims from 1 to 6, characterized by the fact that the axis of said distal portion (16) is curved.

10. A rod as claimed in Claim 9, characterized by the fact that said distal portion (16) presents a bevel (20) on the portion of said surface (18) facing the convex side of said shank (2).

11. A rod as claimed in one of the foregoing Claims, characterized by the fact that the flexibility of said distal portion (16) is greater than that of the rest of said shank (2).

12. A rod as claimed in Claim 10, characterized by the fact that said distal portion (16) presents radial cavities (21).

13. A rod as claimed in Claim 11, characterized by the fact that said distal portion (16) presents longitudinal grooves (22).

14. A rod as claimed in Claim 11, characterized by the fact that said distal portion (16) is made of different material from the rest of said shank (2).

15. A rod as claimed in one of the foregoing Claims from 7 to 14, characterized by the fact that said distal portion (16) is made of composite synthetic material.

16. A rod as claimed in one of the foregoing Claims, characterized by the fact that, in the sagittal plane perpendicular to said front plane, the axis of said proximal portion (14) of said shank (2) forms an angle of 10-30° with the axis of the remaining portion of said shank (2); said two axes blending with each other.

17. A rod as claimed in one of the foregoing Claims, characterized by the fact that the bottom surface (17) of said collar (13) forms an angle of 75-90° with said axis aₚ of said proximal portion (14) of said shank (2).

18. A rod as claimed in one of the foregoing Claims, characterized by the fact that, with the exception of said surface (18) of said distal portion (16), said surface (28) of said shank (2) presents means for assisting osteointegration with the spongy bone (25, 35) of the femur (4).

19. A rod as claimed in Claim 17, characterized by the fact that, with the exception of said surface (18) of said distal portion (16), said surface (28) of said shank (2) presents projections (29) ranging in average size from 0.5 to 1.5 mm.

20. A rod as claimed in Claim 18, characterized by the fact that said projections (29) are also provided on said bottom surface (17) of said collar (13).

21. A rod as claimed in Claim 18, characterized by the fact that, with the exception of said surface (18) of said distal portion (16), said surface (28) of said shank (2) presents a 1-2.5 mm mesh net sheath.

22. A rod as claimed in any one of the foregoing Claims, characterized by the fact that said distal portion (16) of said shank (2) presents a sheath (31) of biodegradable, absorbable material.

23. A rod as claimed in any one of the foregoing Claims, characterized by the fact that said proximal portion (14) presents a coating (30) of hydroxylapatite.

24. A system enabling articulated connection of the periacetabular bone and the femur, characterized by the fact that it comprises:
a femoral rod (1) substantially comprising a shank (2) inserted inside the femur (4); a head (5) extending axially from said shank (2); and a collar (13) projecting transversely from and located at the top end of said shank (2);
an acetabular cup (6) fitted to the periacetabular bone, for housing said head (5) of said rod (1) and enabling angular oscillation of the rod (1) in relation to the periacetabular bone;
a femoral neck (10) projecting integrally from the femur (4), surrounding a portion of said shank (2) adjacent to said top end, and designed to support said collar (13) of said rod (1);
the axial length of the femoral neck (10) being substantially equal to that produced by a resection effected below the head of the femur;
the size of said collar (13) of said rod (1), measured in directions lying in the plane defined by said resection, being substantially equal to the thickness of the femoral neck (10) measured in the same directions.

25. A system as claimed in Claim 24, characterized by the fact that said rod (1) presents the characteristics claimed in Claims 1 to 23.
